# EUROPÄISCHE PATENTSCHRIFT

(11) **EP 0 918 542 B1**
(45) Veröffentlichungstag und Bekanntmachung des Hinweises auf die Patenterteilung: **06.05.2004**
(21) Anmeldenummer: 97934501.4
(22) Anmeldetag: 21.07.1997
(51) Int. Cl.: A61K 47/32

(54) **VERWENDUNG VON (METH)ACRYLSÄURE-COPOLYMEREN ZUR ERHÖHUNG DER PERMEABILITÄT DER SCHLEIMHAUT**
USE OF (METH)ACRYLIC ACID COPOLYMERS TO INCREASE THE PERMEABILITY OF MUCOUS MEMBRANES
UTILISATION DE COPOLYMERES D'ACIDE (METH)ACRYLIQUE POUR AUGMENTER LA PERMEABILITE DE LA MUQUEUSE

(30) Priorität: 01.08.1996 DE 19631084
(43) Veröffentlichungstag der Anmeldung: 02.06.1999
(73) Patentinhaber: BASF AKTIENGESELLSCHAFT, 67056 Ludwigshafen (DE)
(72) Erfinder: KOLTER, Karl, D-67117 Limburgerhof (DE); SUBKOWSKI, Thomas, D-67112 Mutterstadt (DE); RADITSCH, Martin, D-69214 Eppelheim (DE); SCHEHLMANN, Volker, D-67354 Römerberg (DE)
(86) Internationale Anmeldenummer: PCT/EP1997/003899
(87) Internationale Veröffentlichungsnummer: WO 1998/005360

(56) Entgegenhaltungen:
- EP-A- 0 212 641
- EP-A- 0 266 113
- EP-A- 0 386 688
- EP-A- 0 416 842
- EP-A- 0 417 588
- EP-A- 0 490 305
- EP-A- 0 518 468
- EP-A- 0 544 144
- EP-A- 0 682 945
- WO-A-95/15155
- CH-A- 637 017
- US-A- 4 003 991
- HIROYUKI UMEJIMA ET AL: "Preparation and evaluation of Eudragit gels. VIII. Rectal absoprtion of 5-Fluorouracil from Eudispert hv gels in art" JOURNAL OF PHARMACEUTICAL SCIENCES, Bd. 84, Nr. 2, Februar 1995, Seiten 199-202, XP002054265
- BORCHARD G. ET AL: "The potential of mucoadhesive polymers in enhancing intestinal peptide drug absorption. III. Effects of chitosan-glutamate and carbomer on epithelial tight junctions in vitro" JOURNAL OF CONTROLLED RELEASE, Bd. 39, Mai 1996, Seiten 131-138, XP002054266
- HOCHMAN J. ET AL: "Mechanisms of absorption enhancement and tight junction regulation" JOURNAL OF CONTROLLED RELEASE, Bd. 29, 1994, Seiten 253-267, XP002054267
- HOLGADO M.A. ET AL: "Physical characterization of carteolol: Eudragit L binding interaction" INT. J. PHARM., Bd. 114, Nr. 1, 1995, Seiten 13-21, XP002054268

## Beschreibung

Die vorliegende Erfindung betrifft die Verwendung von (Meth)acrylsäure-Ethylacrylat-Copolymeren zur Erhöhung der Permeabilität der Schleimhaut.

Für den parazellulären Transport hydrophiler Wirkstoffe, insbesondere solcher mit höher molekularen Strukturen, bildet das Epithelgewebe eine wichtige Permeationsschranke. Sogenannte "tight junctions" (interzelluläre Verbindungsstellen zwischen benachbarten Epithelzellen, in deren Bereich die Plasma-Membranen unmittelbar aneinanderliegen) gewährleisten, daß das interne Milieu eines Organs vom externen abgeschlossen ist. Die passive parazelluläre Permeabilität dieser Epithelzellen wird bestimmt durch die "Engmaschigkeit" der interzellulären Kontaktpunkte. Eine Aufweitung der "tight junctions" führt zu einer verbesserten Absorption und somit zu einer höheren Bioverfügbarkeit von Wirkstoffen.

Es hat daher in der Vergangenheit nicht an Versuchen gefehlt, Methoden zur Öffnung der interzellulären Kontaktstellen zu entwickeln. Hierbei zeigte sich, daß der Einsatz sowohl oberflächenaktiver Reagentien als auch Ca²⁺- chelatisierender Substanzen vielversprechende Ansatzpunkte lieferte.

Gemäß J. Controlled Rel., 29 (1994) 253, besteht bei Anwendung von oberflächenaktiven Substanzen jedoch die Gefahr einer Zell-Lyse und den damit verbundenen toxischen Nebenwirkungen.

Zahlreiche Publikationen, u.a. in J. Controlled Rel., 36 (1995) 25; Chem. Pharm. Bull. 33 (1985) 4600; The Journal of Cell Biology, 87 (1980) 736 und Int. J. Pharm., 90 (1993) 229 beschreiben den Einfluß von EDTA bzw. EGTA auf die Permeabilität unterschiedlicher Zellsysteme, z.B. von Caco-2-Zellen. Danach kann die Anwesenheit von Ca²⁺-chelatisierenden Substanzen zu einer schnellen aber häufig nicht reversiblen Öffnung der tight junctions führen. Im Falle von EDTA benötigt man außerdem relativ hohe Konzentrationen, um bei neutralem pH-Wert einen Effekt (Reduktion des transepithelialen Widerstandes) zu beobachten. Daneben besteht bei Komplexbildnern mit einem Molekulargewicht ≤ 20 kDa die Gefahr, daß sie systematisch absorbiert werden und so zu ungewünschten toxischen Nebenwirkungen führen können.

In J. Controlled Rel., 29 (1994) 329 konnte gezeigt werden, daß nicht absorbierbare hochmolekulare Verbindungen auf Basis quervernetzter Polyacrylate wie Polycarbophil (Noveon® AA1, B.F. Goodrich) ebenfalls in der Lage sind, tight junctions zu öffnen. Aufgrund ihres extrem hohen Molekulargewichtes (> 1.000 kDa) und ihrer, bereits bei niedrigen Konzentrationen (≥ 0.5 Gew.-%), hohen Viskosität ergeben sich anwendungstechnische Nachteile.

Weiter ist bekannt, daß Polymere mit bioadhäsiven Eigenschaften die Bioverfügbarkeit von Wirkstoffen verbessern können. So wird beispielsweise in EP-A-587 047 die Verwendung von Copolymeren aus (Meth)acrylaten mit verschiedenen ungesättigten Carbonsäuren für pharmazeutische Zubereitungen auf Gestagen-Basis beschrieben.

Die Bioverfügbarkeit schwerlöslicher Wirkstoffe läßt sich nach EP-B-410 422 dadurch erhöhen, daß sie nach der Formulierung durch den Einfluß von (Meth)acrylsäure/(Meth)acrylat-Copolymeren amorph vorliegen und somit besser löslich sind.

Es war nun Aufgabe der Erfindung, Polymere zu finden, die reversibel die Permeabilität von Epithelzellen erhöhen, ohne dabei die oben genannten anwendungstechnischen Nachteile aufzuweisen oder Toxizitätsprobleme zu verursachen.

Überraschenderweise wurde gefunden, daß durch Verwendung von (Meth)acrylsäure-Ethylacrylat-Copolymeren eine Erhöhung der Permeabilität der Schleimhaut und damit eine Steigerung der Permeation von Wirkstoffen erzielt wird.

Die erfindungsgemäßen Copolymere enthalten als Monomere Acrylsäure bzw. Methacrylsäure, die in Form ihrer freien Säure, ihrer Salze und/oder ihrer Anhydride eingesetzt werden können.

Werden die Monomere in Form ihrer Salze zur Polymerisation verwendet, so sind die Erdalkali-, Alkali- oder Ammoniumsalze oder die Salze organischer Amine bevorzugt, besonders bevorzugt sind die Alkali- oder Ammoniumsalze.

Als weiteres Comonomer ist Ethylacrylat enthalten.

Das (Meth)acrylsäure-Ethylacrylat-Comonomer-Molverhältnis kann im erfindungsgemäßen Copolymer breit variiert werden. Beispielsweise liegt es im Bereich von 99:1 bis 1:99, bevorzugt von 70:30 bis 30:70.

Üblicherweise werden die Copolymere in Form von pharmazeutischen Zubereitungen zusammen mit dem Wirkstoff verabreicht. Als pharmazeutische Darreichungsform kommen Tabletten, Extrudate, Granulate, Pellets, Pulver, Kapseln, Suppositorien, Salben, Suspensionen oder Emulsionen in Betracht, wobei die Verabreichung je nach Anwendungsgebiet oral, sublingual, buccal, rectal, pulmonal, nasal oder über die Augenschleimhaut erfolgen kann. Bevorzugte Darreichungsformen sind a) Matrixtabletten, b) Manteltabletten sowie c) Filmtabletten. Insbesondere Matrixtabletten, in denen das erfindungsgemäße Polymer und der Wirkstoff nach inniger Durchmischung zusammen verpreßt werden, stellen eine Darreichungsform mit hohem Wirkpotential dar. In diesen Darreichungsformen ist der Anteil an erfindungsgemäß zu verwendendem Copolymer im allgemeinen größer als 50 Gew.-%, insbesondere im Bereich von 60 bis 99 Gew.-%, vorzugsweise liegt er zwischen 75 und 99 Gew.-%, jeweils bezogen auf die Gesamtmasse der Darreichungsform. Es ist aber auch möglich, zunächst die Schleimhaut z.B. Darm-, Rachen- oder Augenschleimhaut mit dem permeabilitätssteigernden Copolymer zu behandeln und anschließend den pharmazeutischen Wirkstoff zu verabreichen.

Die oben genannten Darreichungsformen werden in der Regel unter Zusatz von Füll-, Binde-, Spreng-, Gleitmitteln oder anderen Hilfsstoffen hergestellt. Als Füllstoffe und Trockenbindemittel für Tabletten dienen u.a. Lactose, Saccharose, Mannit, Sorbit, mikrokristalline Cellulose, Stärke, Dicalciumphosphat und Polyglycole. Geeignete Bindemittel für die Granulierung sind z.B. Stärke, Alginate, Polyvinylpyrrolidon und Carboxymethylcellulose. Geeignete Fließregulierungsmittel sind z.B. Stärke, Talkum und Siliciumdioxid. Als Schmiermittel bei der maschinellen u.a. Stärke, Cellulosederivate sowie quervernetztes Polyvinylpyrrolidon.

Je nach Anwendungsgebiet und Wirkstoff werden die erfindungsgemäßen Copolymere vorteilhafterweise in neutralisierter, teilneutralisierter oder nichtneutralisierter Form verwendet. Liegen die Copolymere in nichtneutralisierter Form vor, so ist die Anwesenheit einer Base oder eines Protonenakzeptors, der entweder aus einem weiteren Hilfsstoff und/oder direkt aus dem Wirkstoff besteht, oftmals vorteilhaft.

Im Falle eines basischen Wirkstoffs kann dieser ganz oder teilweise in Salzform mit dem erfindungsgemäßen Copolymer vorliegen.

Die Herstellung der Copolymere erfolgt nach den in der Literatur bekannten Verfahren wie beispielsweise Lösungsmittelpolymerisation, Suspensionspolymerisation oder Emulsionspolymerisation, wobei die Emulsionspolymerisation bevorzugt ist.

Die Emulsionspolymerisation erfolgt in üblicher Weise unter Verwendung von Initiatoren, wie Peroxo- oder Azoverbindungen, beispielsweise Dibenzoyloxid, t-Butylperpivalat, t-Butylper-2-ethylhexanoat, Di-t-butylperoxid, t-Butylhydroperoxid, Alkalimetall- oder Ammoniumpersulfate, Azo-bis-isobutyronitril, 2,2'-Azo-bis-(2-methylbutyronitril), 2,2'-Azo-bis-(2,4-dimethylvaleronitril), 1,1'-Azo-bis-(1-cyclohexancarbonitril), 2,2'-Azobis-(2-amidinopropan)salze, 4,4'-Azo-bis-(4-cyanovaleriansäure) oder 2-(Carbamoylazo)-isobutyronitril etc., Wasserstoffperoxid oder Redoxinitiatoren. Die Initiatoren werden üblicherweise in Mengen bis zu 10, vorzugsweise 0.02 bis 5 Gew.-%, bezogen auf die zu polymerisierenden Monomere eingesetzt.

Die Polymerisation wird in Anwesenheit eines für diese Zwecke üblichen Emulgators und/oder Schutzkolloids durchgeführt. Geeignete Schutzkolloide sind beispielsweise Polyvinylalkohole, Cellulosederivate oder Polyvinylpyrrolidone. Die Emulgatoren können anionischer, kationischer oder nicht-ionischer Natur sein. Geeignete Emulgatoren sind beispielsweise ethoxylierte Mono-, Diund Trialkylphenole, ethoxylierte Fettalkohole oder Sorbitanester, Alkali- und Ammoniumsalze von Alkylsulfaten oder Alkylethersulfaten, von Alkylsulfonsäuren, von Ligninsulfonsäure und von Alkylarylsulfonsäuren oder Alkyldiphenyloxidsulfonate.

Die Emulsionspolymerisation erfolgt üblicherweise unter Sauerstoffausschluß bei Temperaturen im Bereich von 20 bis 200°C. Die Polymerisation kann diskontinuierlich oder kontinuierlich durchgeführt werden.

Vorzugsweise dosiert man zumindest einen Teil der Monomere, Initiatoren und gegebenenfalls Regler, wie Aldehyde, Halogenverbindungen oder Schwefelverbindungen, beispielsweise Formaldehyd, Acetaldehyd, Bromtrichlormethan, Mercaptoethanol, Thioglykolsäure oder Dodecylmercaptan, während der Polymerisation gleichmäßig in das Reaktionsgefäß. Die Monomeren und der Initiator können jedoch auch im Reaktor vorgelegt und polymerisiert werden, wobei gegebenenfalls gekühlt werden muß.

Auf diese Weise werden Copolymere mit einem mittleren Molekulargewicht (Gewichtsmittel) von 50.000 bis 2 Mio. erhalten. Für die erfindungsgemäße Verwendung sind Copolymere mit einem mittleren Molekulargewicht von 20.000 bis 1.500.000, insbesondere 120.000 bis 400.000 besonders geeignet.

Für die Trocknung der Polymer-Dispersion kommen alle üblichen Technologien wie beispielsweise die Dünnschichttrocknung, die Sprühwirbelschichttrocknung, die Sprühtrocknung oder die Gefriertrocknung in Frage.

Der Nachweis von permeabilitätssteigernden Eigenschaften der erfindungsgemäß zu verwendenden Polymere erfolgte in einem in vitro Testsystem am Beispiel von Caco-2-Zellkulturen (Fa. ATCC, Pockville, Maryland, USA). Diese Zellinie bildet auf Polycarbonatmembranen einen Gewebeverband, der dem Endothel des Dünndarmes in Aussehen und Proteinzusammensetzung sehr ähnlich ist. Im Transwellsystem (Fa. Costar, Bodenheim, Deutschland) trennt dieses künstliche Endothelgewebe zwei mit Medium gefüllte Kammern in das apikale und das basolaterale Kompartiment. Dieser Aufbau ermöglicht die Bestimmung der Peremeation einer Substanz durch die Caco-2-Gewebeschicht. Dabei zeigen sowohl der transepitheliale Widerstand (TEER) als auch die Permeation fluoreszierender Marker [z.B. Fluorescein-Isothiocyanat-Dextran (FITC-Dextran-4400; FITC-Dextran-11000); Fluorescein-Biotin = (Biotinamidocaproylamido)-(pentylthioureidyl)-Fluorescein] durch die Zell-Monoschicht an, inwieweit die tight junctions geöffnet bzw. geschlossen sind. Eine Permeabilitätsverbesserung zeigt sich durch ein reversibles Öffnen der tight junctions an.

### Versuchsbeispiele:

### 1. Aufzucht der Zellkultur

Caco-2-Zellen wurden bei 37°C in DMEM (Dulbeccos Modified Eagle's Medium, Fa. Gibco BRL, Eggenstein, Deutschland) mit 10% FCS (Foetales Kälberserum), 1% L-Glutamin und 4.5 mg/ml Glucose bei 5% CO₂ und 95% relativer Luftfeuchtigkeit kultiviert. Die Zellen wurden nach trypsinieren in einer Dichte von 3x10⁵ Zellen/cm² auf Transwell Polycarbonatfilter ausgesät. Zur Monolayerbildung wurden Zellen zwischen der dreißigsten und fünfzigsten Passage herangezogen. Die Monolayer wurden nach 15 bis 21 Tagen (Mediumwechsel: alle drei Tage) nach dem Aussäen der Zellen im Experiment eingesetzt.

### 2. Bestimmung der Permeationsgeschwindigkeit

Vor dem Experiment wurde das Caco-2-Zellgewebe in den Transwell-Filtereinsätzen mit PBS (Phosphate buffer saline) gewaschen. Danach wurde das apikale Kompartiment mit EBSS (Earl's gepufferter Salzlösung, pH 6), das basolaterale Kompartiment mit DMEM aufgefüllt. Nach Zugabe des zu untersuchenden, auf pH 7 neutralisierten Polymers [Kollicoat® MAE 30D (Ethylacrylat/Methacrylsäure 1:1, BASF), Eudragit® L100 (Methacrylsäure/Methacrylsäuremethylester 1:1, Röhm), Eudragit® S100 (Methacrylsäure/Methacrylsäuremethylester 1:2, Röhm)] in einer Konzentration von 0.5 bis 3 Gew.-% und der Markersubstanz in das apikale Kompartiment wurden nach 24 Stunden Proben entnommen und die Permeationsgeschwindigkeit der Markersubstanz bestimmt.

Die Analytik der Versuche erfolgte mittels Fluoreszenz-Spektrophotometrie (Spex Fluorolog 1680: Anregungswellenlänge: 488 nm, Emissionswellenlänge: 513 nm) sowie im Falle von C₂₂H₃₂N₆O₄ mittels HPLC (Säulenmaterial: Vidac C-18; Laufmittel: Wasser/Acetonitril/0.1% Trifluoressigsäure; Detektion: 240 nm). Die Versuchsergebnisse sind in Tabelle 1 zusammengestellt. Nicht durchgeführte Messungen sind durch "-" gekennzeichnet worden.

**Tabelle 1:**

| | Kontrolle | Kollicoat® MAE 30 D | | | |
|---|---|---|---|---|---|
| | | 0.5% | 1.0% | 1.5% | 3.0% |

| | Permeationsgeschwindigkeit [(cm/sec)*10⁻⁸] | | | | |
|---|---|---|---|---|---|
| Testsubstanz | | | | | |
| Fluorescein-Biotin | 2.3 | 2.3 | 5.8 | 20 | - |
| Dextran-FITC 4400 | 0.8 | - | 9.6 | - | 935 |
| Dextran-FITC 11000 | 2.3 | - | 17.0 | - | 769 |
| C₂₂H₃₂N₆O₄ | 56 | 79 | 94 | 280 | - |
| | | | | | |

| | Kontrolle | Eudragit® L 100 | | | |
|---|---|---|---|---|---|
| | | | 2.0% | | |

| | Permeationsgeschwindigkeit [(cm/sec).10⁻⁸] | | | | |
|---|---|---|---|---|---|
| Testsubstanz | | | | | |
| Fluorescein-Biotin | 2.3 | | 4.8 | | |
| C₂₂H₃₂N₆O₄ | 56 | | 104 | | |
| | | | | | |
| | | | | | |

| | Kontrolle | Eudragit® S 100 | | | |
|---|---|---|---|---|---|
| | | | 2.0% | | |

| | Permeationsgeschwindigkeit [(cm/sec).10⁻⁸] | | | | |
|---|---|---|---|---|---|
| Testsubstanz | | | | | |
| Fluorescein-Biotin | 2.3 | | 7.1 | | |
| C₂₂H₃₂N₆O₄ | 56 | | 103 | | |
| | | | | | |

C₂₂H₃₂N₆O₄ = HOOC-CH₂-(D)-Cha-Pro-NH-3-(6-Am)-Pico

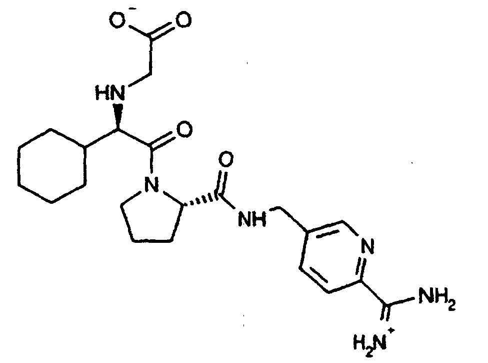

Wie aus Tabelle 1 zu entnehmen ist, zeigten alle drei untersuchten Polymere permeationsverstärkende Eigenschaften. Im vergleich zum Kontrollversuch führte der Einsatz von Kollicoat® MAE 30D schon ab einer Konzentration von 1% zu einer deutlichen Permeationssteigerung.

### 3. Bestimmung des transepithelialen Widerstandes (TEER) :

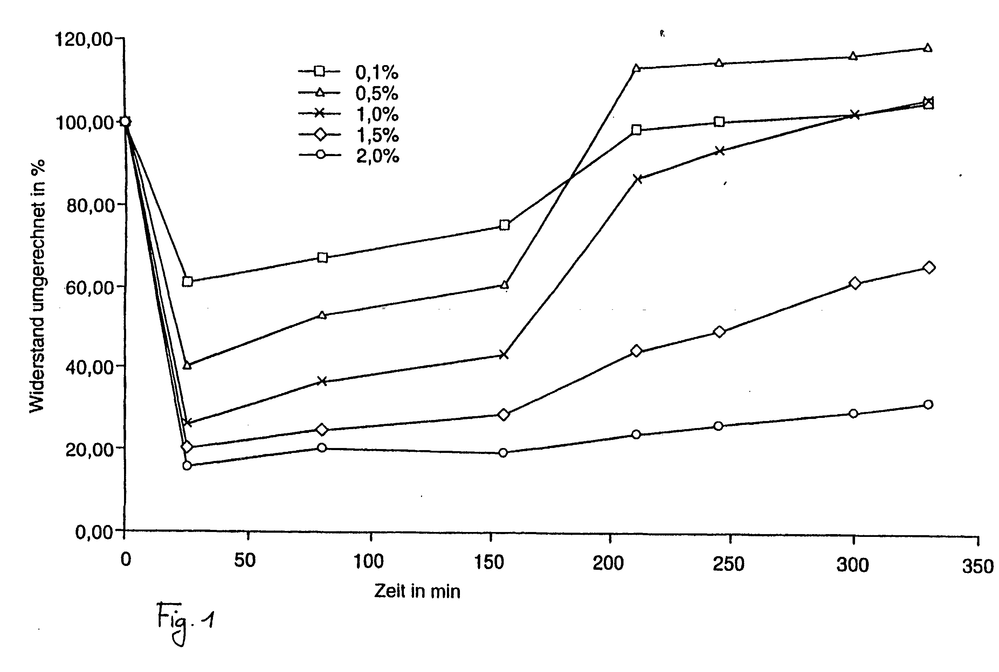

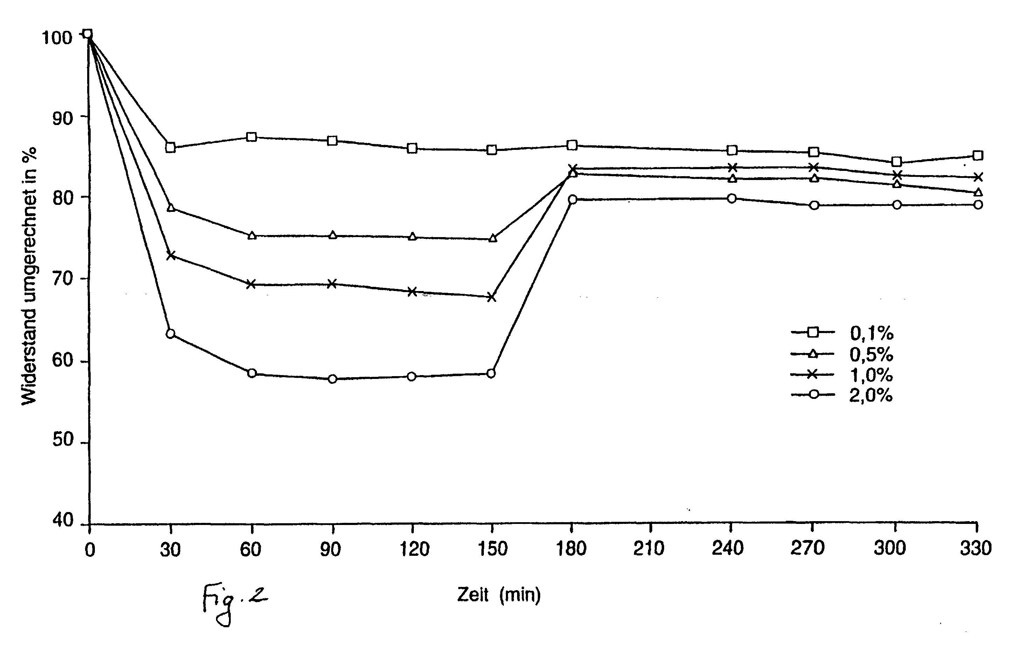

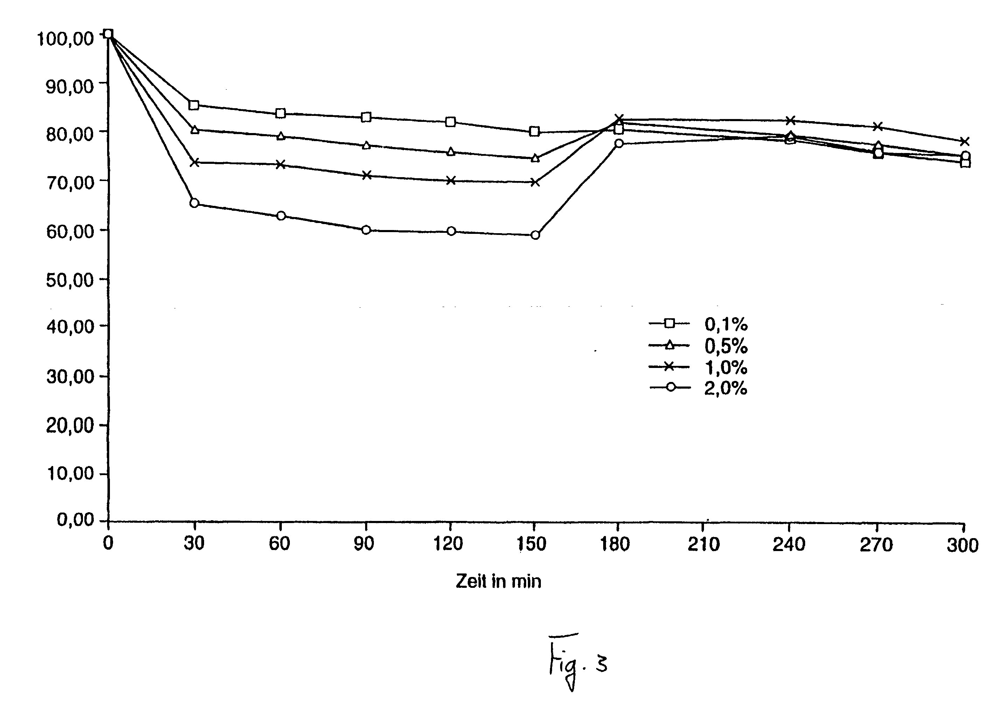

Die Wirkung der erfindungsgemäßen Polymere auf die Dichtigkeit des Gewebes (Öffnung der tight junctions) wurde durch Messung des transepithelalen elektrischen Widerstandes bestimmt. Nach Waschen des Caco-2-Zellgewebes mit PBS wurde das apikale Kompartiment mit EBSS (pH 6) und das basolaterale Kompartiment mit DMEM aufgefüllt. Vor und nach der Zugabe des auf pH 7 neutralisierten Polymers in unterschiedlichen Konzentrationen (0.1 bis 2%) in das apikale Kompartiment wurde der TEER zu verschiedenen Zeitpunkten bestimmt (Fig.1: Kollicoat® MAE 30D; Fig.2: Eudragit® L100; Fig.3: Eudragit® S100). Nach 150 Minuten wurde die Lösung gegen EBSS, pH 6.0, ausgetauscht und die Regenerierung des Gewebes anhand des TEER untersucht.

Sowohl Eudragit® S bzw. L100 als auch Kollicoat® MAE 30D insbesondere führten bei den Messungen zu einem deutlichen Abfall des transepithelialen Widerstandes der zellulären Monoschichten von Caco-2-Zellen.

### 4. Beispiele für pharmazeutische Darreichungsformen

### a. Atenolol-Filmtabletten

| | |
|---|---|
| Atenolol | 50 mg |
| Ludipress® | 50 mg |
| Methacrylsäure-Ethylacrylat Copolymer (1:1), neutralisiert auf pH 7 | 146,5 mg |
| Aerosil 200® | 2 mg |
| Magnesiumstearat | 1,5 mg |
| Tablettenmasse | 250 mg |

1000 g Atenolol, 1000 g Ludipress® (BASF), 2930 g Methacrylsäure-Ethylacrylat-Copolymer neutralisiert auf pH 7, 40 g Aerosil 200® (Degussa) und 30 g Magnesiumstearat wurden über ein 0,8 mm Sieb vorgesiebt und in einem Turbula-Mischer 5 min. gemischt. Anschließend wurden auf einer Rundläufertablettenpresse bei einer Preßkraft von 20 kN gewölbte Tabletten mit einem Durchmesser von 9 mm hergestellt.

Auf diese gewölbten Tabletten wurde in einem Horizontaltrommelcoater des Typs Accela-Cota (Fa. Manesty) bei einer Zuluft-temperatur von 50 °C ein Filmüberzug mit Methacrylsäure-Ethylacrylat-Copolymer aufgebracht. Die Filmcoatingdispersion wies folgende Zusammensetzung auf:

| | |
|---|---|
| Titandioxid | 0,5 Gew.-% |
| Talkum | 2 Gew.-% |
| Sicovit® Rot 30 | 0,5 Gew.-% |
| Kollidon® 30 | 0,5 Gew.-% |
| Methacrylsäure-Ethylacrylat Copolymer (1:1), neutralisiert auf pH 7 | 15 Gew.-% |
| Triethylcitrat | 1,5 Gew.-% |
| Wasser | 80 Gew.-% |

Die Auftragsmenge für 5000 g gewölbte Tabletten betrug 1680,8 g Filmcoatingdispersion.

### b. Propranolol-Tabletten

| | |
|---|---|
| Propranolol-HCl | 160 mg |
| Methacrylsäure-Ethylacrylat Copolymer (1:1), neutralisiert auf pH 7 | 190 mg |
| Aerosil 200® | 3,4 mg |
| Magnesiumstearat | 1,6 mg |
| Tablettenmasse | 355 mg |

1600 g Propranolol-HCl, 1900 g Methacrylsäure-Ethylacrylat-Copolymer, neutralisiert auf pH 7, 34 g Aerosil 200® und 16 g Magnesiumstearat wurden über ein 0,8 mm-Sieb vorgesiebt und in einem Turbulamischer 5 min. gemischt. Die Verpressung erfolgte auf einer Rundläufertablettenpresse des Typs Korsch PH106 bei einer Preßkraft von 20 kN und einer Geschwindigkeit von 30 Umdrehungen/min. Es wurden biplane, facettierte Tabletten mit 10 mm Durchmesser und 355 mg Gewicht hergestellt.

### c. Furosemid-Mikrotabletten

| | |
|---|---|
| Furosemid | 1 mg |
| Methacrylsäure-Ethylacrylat Copolymer (1:1), neutralisiert auf pH 7 | 5,95 mg |
| Magnesiumstearat | 0,05 mg |
| Tablettenmasse | 7 mg |

100 g Furosemid und 595 g Methacrylsäure-Ethylacrylat-Copolymer neutralisiert auf pH 7 wurden in einem Stephan-Mischer gemischt und unter Rühren mit 133 g Isopropanol befeuchtet. Die feuchte Masse wurde durch ein Sieb mit einer Maschenweite von 0,6 mm gedrückt und auf einer Horde in dünner Schicht 24 h bei Raumtemperatur getrocknet. Das trockene Granulat wurde über ein 0,8 mm-Sieb gegeben mit ebenfalls gesiebtem Magnesiumstearat versetzt und im Turbula-Mischer 5 min. gemischt und nochmals über ein 0,8 mm-Sieb gegeben. Auf einer Excenterpresse des Typs Korsch EKO wurden danach gewölbte Mikrotabletten mit 2 mm Durchmesser und ca. 2 mm Höhe hergestellt. Zur Erreichung einer Dosierung von 40 mg wurden jeweils 40 Mikrotabletten in Gelatinesteckkapseln gefüllt.

### d. Methyldopa-Tabletten

| | |
|---|---|
| Methyldopa | 250 mg |
| Methacrylsäure-Ethylacrylat Copolymer (1:1), neutralisiert auf pH 7 | 347,5 mg |
| Aerosil 200® | 3,5 mg |
| Kollidon® CL | 6 mg |
| Magnesiumstearat | 3 mg |
| Tablettenmasse | 610 mg |

2500 g Methyldopa, 3475 g Methacrylsäure-Ethylacrylat-Copolymer, 35 g Aerosil 200, 60 g Kollidon® CL (BASF) und 30 g Magnesiumstearat wurden über ein 0,8 mm-Sieb vorgesiebt und in einem Turbulamischer 5 min. gemischt. Anschließend wurde diese Pulvermischung auf einer Rundläufertablettenpresse bei einer Preßkraft von 30 kN und einer Geschwindigkeit von 30 Umdrehungen/min. zu biplanen, facettierten Tabletten mit einem Durchmesser von 12 mm verpreßt.

### e. S-Adenosylmethionin-Lutschtabletten

| | |
|---|---|
| S-Adenosylmethionin | 100 mg |
| Methacrylsäure-Ethylacrylat Copolymer (1:1), neutralisiert auf pH 7 | 700 mg |
| Mannit | 200 mg |
| Aspartame | 3 mg |
| Orangenaroma | 5 mg |
| Kollidon® VA 64 | 37 mg |
| Aerosil 200® | 5 mg |
| Magnesiumstearat | 5 mg |
| Tablettenmasse | 1055 mg |

500 g S-Adenosylmethionin, 3500 g Methacrylsäure-Ethylacrylat-Copolymer neutralisiert auf pH 7, 1000 g Mannit, 15 g Aspartame, 25 g Orangenaroma, 25 g Aerosil 200 und 185 g Kollidon® VA 64 wurden über ein 0,8 mm-Sieb vorgesiebt und im Turbula-Mischer 5 min. gemischt. Anschließend wurden 25 g Magnesiumstearat, das zuvor über ein 0,5 mm-Sieb gesiebt wurde, zugegeben und in 2,5 min. untergemischt. Auf einer Rundläufertablettenpresse wurden bei einem Preßdruck von 35 kN und einer Umdrehungsgeschwindigkeit von 30 U/min. biplane, facettierte Tabletten mit 1055 mg Gewicht hergestellt.

## Patentansprüche

1. Verwendung von (Meth)acrylsäure-Ethylacrylat-Copolymeren zur Herstellung von Arzneimitteln zur Erhöhung der Permeabilität der Schleimhaut.

2. Verwendung nach Anspruch 1 **dadurch gekennzeichnet, daß** das (Meth)acrylsäure:Comonomer-Molverhältnis von 99:1 bis 1:99 variiert.

3. Verwendung gemäß den Ansprüchen 1 oder 2, **dadurch gekennzeichnet, daß** die Arzneimittel zur Verabreichung in oraler, fester Form geeignet sind.

## Claims

1. The use of (meth)acrylic acid/ethyl acrylate copolymers for producing medicaments for increasing mucosal permeability.

2. The use as claimed in claim 1, wherein the (meth)acrylic acid: comonomer molar ratio varies from 99:1 to 1:99.

3. The use as claimed in claims 1 or 2, wherein the medicaments are suitable for administration in oral solid form.

## Revendications

1. Utilisation de copolymères acide (méth)acrylique-acrylate d'éthyle pour la préparation de médicaments prévus pour accroître la perméabilité des muqueuses.

2. Utilisation selon la revendication 1, **caractérisée en ce que** le rapport molaire acide (méth)acrylique/comonomère va de 99 : 1 à 1 : 99.

3. Utilisation selon la revendication 1 ou 2, **caractérisée en ce que** le médicament est prévu pour l'administration sous forme solide par voie orale.
